# EUROPEAN PATENT APPLICATION

(11) **EP 4 706 582 A2**
(43) Date of publication of application: **11.03.2026**
(21) Application number: 26153837.5
(22) Date of filing: 08.01.2021
(51) Int. Cl.: A61B 90/00

(54) **VASCULAR MONITORING COLLAR**

(30) Priority: 10.01.2020 US 202062959587 P; 11.06.2020 US 202063037772 P
(62) Divisional of application: 21709158.6
(71) Applicant: BAXTER INTERNATIONAL INC., Deerfield, IL 60015-4634 (US); BAXTER HEALTHCARE SA, 8152 Glattpark (Opfikon) (CH)
(72) Inventor: GROFF, Patti, Ann, Deerfield, IL 60015 (US); STUDER, James, Douglas, Deerfield, IL 60015 (US); MCPEAK, Daniel, Robert, Deerfield, IL 60015 (US); COTHREL, Grant, Robert, Deerfield, IL 60015 (US); KO, Benjamin, Lee, Deerfield, IL 60015 (US); WITKOWSKI, Shannon, M., Deerfield, IL 60015 (US); RIZZONI, Maria, Caterina, Deerfield, IL 60015 (US)
(74) Representative: SONN Patentanwälte GmbH & Co KG

(57) **Abstract**

A vascular monitoring system comprising a strap configured to be positioned about a patient's vessel and a transducer coupled to the strap. The transducer is configured to emit an ultrasonic signal that is transmitted through the patient's vessel. The strap includes multiple sizing holes that are spaced along the strap and are adapted to maintain the strap in a closed configuration when fit over a closure prong.

## Description

### RELATED APPLICATIONS

This application claims the benefit of and priority to U.S. Provisional Patent Application No. 62/959,587 filed January 10, 2020, entitled "VASCULAR MONITORING COLLAR" and U.S. Provisional Patent Application No. 63/037,772 filed June 11, 2020 entitled "VASCULAR MONITORING COLLAR," which are both incorporated herein by reference in their entirety.

### BACKGROUND

Plastic and reconstructive surgery regularly uses free flaps, for example in breast reconstruction. In free flap tissue surgery, a free flap (e.g., tissue and/or muscle and its associated artery and vein) is removed from one part of the body or donor site and is reattached to another part of the body or recipient site. The artery and vein of the transferred tissue and/or muscle are then anastomosed to a native artery and vein in order to achieve blood circulation in the transferred free flap (e.g., tissue and/or muscle).

The anastomosis of the free flap tissue to the native tissue is typically done using microvascular techniques, including under microscopic visualization. In previous years, several surgical instruments and techniques have been developed to aid in anastomosis. One known system for creating an anastomosis is an anastomosis coupler, described in U.S. Pat. No. 7,192,400, the disclosure of which is incorporated herein by reference. This anastomotic coupler is a surgical instrument that allows a surgeon to more easily and effectively join together two blood vessel ends. The coupler involves the use of two fastener portions, in the shape of rings, upon which are secured respective sections of the vessel to be attached. Each fastener portion is also provided with a series of pins, and corresponding holes for receiving those pins, in order to close and connect the portions, and in turn the vessel, together.

While free flap surgeries have a history of success, highly undesirable consequences of a flap failure still remain a possibility. One of the main causes of flap failure is a lack of blood being supplied to the flap tissue after the free flap is reattached at the recipient site. Things that commonly disturb circulation in a flap include vascular occlusion, hemorrhage, or infection. When not enough blood is supplied to the flap tissue, tissue necrosis results. However, if it can be recognized early enough that the flap is not receiving adequate circulation, it may be saved, or salvaged. The window of time for salvaging the flap after a lack of blood flow is recognized is very small. It is therefore critical that any lack of blood flow in a transferred flap be quickly recognized.

Handheld Doppler probes, which are typically permanently positioned on the distal tip of a pen-like device instead of being placed or left within the body, are helpful in blood flow monitoring, but they suffer from several drawbacks. One drawback with handheld probes is their inability to be reliably positioned about a vessel.

It is of great importance after microvascular surgery to monitor the region of the surgery in order to make sure that the blood flow is maintained at the desired level and that no problems, such as thromboses have occurred. Should thrombosis occur, the transferred tissue would die. Other indirect means of monitoring the functioning of blood flow through blood vessels, which have been subjected to microvascular surgery, are also often inadequate. For example, surface temperature measurements, transcutaneous PO₂ monitoring, photo plethysmography and laser Doppler flow meters have been employed. However, these approaches generally require an accessible exposed portion of the flap. Additionally, buried free tissue transfers and intraoral flaps cannot be monitored effectively by these methods.

### SUMMARY

The present disclosure provides improved vascular monitoring straps and collars, which may be used with vascular monitoring systems, devices and methods to improve the accessibility, detection and/or reliability of detecting blood flow to confirm vessel patency at an anastomotic site.

Aspects of the subject matter described herein may be useful alone or in combination with one or more other aspects described herein. In a first exemplary aspect of the present disclosure a vascular monitoring system includes a collar configured to be positioned about a patient's vessel and a transducer coupled to the collar. The transducer is configured to emit an ultrasonic signal that is transmitted through the patient's vessel.

In accordance with another exemplary aspect of the present disclosure, which may be used in combination with any one or more of the preceding aspects, the collar includes at least one eyelet that is adapted to be sutured to adjacent tissue to fixedly position the collar about the patient's vessel.

In accordance with another exemplary aspect of the present disclosure, which may be used in combination with any one or more of the preceding aspects, the collar includes a probe holder sized and shaped to receive the transducer.

In accordance with another exemplary aspect of the present disclosure, which may be used in combination with any one or more of the preceding aspects, the transducer is coupled to the collar through a friction fit with the probe holder.

In accordance with another exemplary aspect of the present disclosure, which may be used in combination with any one or more of the preceding aspects, the collar is made of at least one of implant grade liquid-silicon rubber ("LSR") and high-consistency silicone rubber ("HCR") with a durometer between 40 and 80.

In accordance with another exemplary aspect of the present disclosure, which may be used in combination with any one or more of the preceding aspects, the collar is configured to be positioned about an anastomosis site of the patient's vessel.

In accordance with another exemplary aspect of the present disclosure, which may be used in combination with any one or more of the preceding aspects, the collar is configured to be positioned at a location that is one of upstream of an anastomosis site of the patient's vessel and downstream of the anastomosis site of the patient's vessel.

In accordance with another exemplary aspect of the present disclosure, which may be used in combination with any one or more of the preceding aspects, the transducer is removably coupled to the collar.

Aspects of the subject matter described herein may be useful alone or in combination with one or more other aspects described herein. In a second exemplary aspect of the present disclosure a vascular collar includes a cylindrical body portion with an opening, the opening having an inside diameter sized and shaped to be positioned about a patient's vessel. The vascular collar also includes a probe holder and at least one mounting tab. The probe holder is configured to receive a transducer.

In accordance with another exemplary aspect of the present disclosure, which may be used in combination with any one or more of the preceding aspects, the inside diameter is between 1.0mm and 4.0mm.

In accordance with another exemplary aspect of the present disclosure, which may be used in combination with any one or more of the preceding aspects, the transducer is configured to emit an ultrasonic signal that is transmitted through the patient's vessel.

Aspects of the subject matter described herein may be useful alone or in combination with one or more other aspects described herein. In a third exemplary aspect of the present disclosure a vascular monitoring system includes a collar configured to be positioned about a patient's vessel. The collar is configured to transition from an open configuration to a closed configuration. The vascular monitoring system also includes a transducer coupled to the collar. The transducer is configured to emit an ultrasonic signal that is transmitted through the patient's vessel.

In accordance with another exemplary aspect of the present disclosure, which may be used in combination with any one or more of the preceding aspects, the collar includes at least one closure structure configured to maintain the collar in the closed configuration.

In accordance with another exemplary aspect of the present disclosure, which may be used in combination with any one or more of the preceding aspects, the at least one closure structure includes a first eyelet and a second eyelet.

In accordance with another exemplary aspect of the present disclosure, which may be used in combination with any one or more of the preceding aspects, the at least one closure structure is adapted to be sutured to adjacent tissue to fixedly position the collar about the patient's vessel.

In accordance with another exemplary aspect of the present disclosure, which may be used in combination with any one or more of the preceding aspects, the collar includes a probe holder sized and shaped to receive the transducer.

In accordance with another exemplary aspect of the present disclosure, which may be used in combination with any one or more of the preceding aspects, the transducer is coupled to the collar through a friction fit with the probe holder.

In accordance with another exemplary aspect of the present disclosure, which may be used in combination with any one or more of the preceding aspects, the collar is made of at least one of implant grade liquid-silicon rubber ("LSR") and high-consistency silicone rubber ("HCR") with a durometer between 40 and 80.

In accordance with another exemplary aspect of the present disclosure, which may be used in combination with any one or more of the preceding aspects, the collar is configured to be positioned about an anastomosis site of the patient's vessel.

In accordance with another exemplary aspect of the present disclosure, which may be used in combination with any one or more of the preceding aspects, the collar is configured to be positioned at a location that is one of upstream of an anastomosis site of the patient's vessel and downstream of the anastomosis site of the patient's vessel.

In accordance with another exemplary aspect of the present disclosure, which may be used in combination with any one or more of the preceding aspects, the transducer is removably coupled to the collar.

Aspects of the subject matter described herein may be useful alone or in combination with one or more other aspects described herein. In a fourth exemplary aspect of the present disclosure a vascular collar includes a body portion that is configured to transition from an open configuration to a closed configuration. The body portion has an opening in the closed configuration, and the opening has an inside diameter sized and shaped to be positioned about a patient's vessel. The vascular collar also includes a probe holder and at least one mounting tab. The probe holder is configured to receive a transducer.

In accordance with another exemplary aspect of the present disclosure, which may be used in combination with any one or more of the preceding aspects, the mounting tab includes a closure feature that is adapted to retain the collar in the closed configuration after transitioned from the open configuration to the closed configuration.

In accordance with another exemplary aspect of the present disclosure, which may be used in combination with any one or more of the preceding aspects, the body portion is made from a flexible/malleable material that allows the body portion to transition from the open configuration to the closed configuration when a closure force is applied to the collar.

Aspects of the subject matter described herein may be useful alone or in combination with one or more other aspects described herein. In a fifth exemplary aspect of the present disclosure a vascular monitoring system includes a strap configured to be positioned about a patient's vessel, a clasp configured to maintain the strap in a closed configuration about the patient's vessel, and a transducer coupled to the strap. The transducer is configured to emit an ultrasonic signal that is transmitted through the patient's vessel.

In accordance with another exemplary aspect of the present disclosure, which may be used in combination with any one or more of the preceding aspects, the strap includes at least one eyelet that is adapted to be sutured to adjacent tissue to fixedly position the strap about the patient's vessel.

In accordance with another exemplary aspect of the present disclosure, which may be used in combination with any one or more of the preceding aspects, the strap includes a probe holder sized and shaped to receive the transducer.

In accordance with another exemplary aspect of the present disclosure, which may be used in combination with any one or more of the preceding aspects, the transducer is coupled to the strap through a friction fit with the probe holder.

In accordance with another exemplary aspect of the present disclosure, which may be used in combination with any one or more of the preceding aspects, the strap is made of at least one of at least one of implant grade liquid-silicon rubber ("LSR"), high-consistency silicone rubber ("HCR"), high density polyethylene ("HDPE"), Nusil 4750, Nusil 4840, and a thermoplastic.

In accordance with another exemplary aspect of the present disclosure, which may be used in combination with any one or more of the preceding aspects, the strap, in its closed configuration, is configured to be positioned about an anastomosis site of the patient's vessel.

In accordance with another exemplary aspect of the present disclosure, which may be used in combination with any one or more of the preceding aspects, the strap, in its closed configuration, is configured to be positioned at a location that is one of upstream of an anastomosis site of the patient's vessel and downstream of the anastomosis site of the patient's vessel.

In accordance with another exemplary aspect of the present disclosure, which may be used in combination with any one or more of the preceding aspects, the transducer is removably coupled to the collar.

Aspects of the subject matter described herein may be useful alone or in combination with one or more other aspects described herein. In a sixth exemplary aspect of the present disclosure a vascular strap includes an elongate strap body having a first end and second end, a plurality of sizing holes positioned along the strap body starting near the first end, and a closure prong positioned adjacent the second end of the strap body. The closure prong is sized and shaped to press-fit through a sizing hole of the plurality of sizing holes, and the closure prong is configured to maintain the vascular strap in a closed configuration when press-fit through the sizing hole. The closed configuration forms a cylindrical shape having an inside diameter sized and shaped to be positioned about a patient's vessel. Additionally, the vascular strap includes a probe holder and at least one mounting tab. The probe holder configured to receive a transducer.

In accordance with another exemplary aspect of the present disclosure, which may be used in combination with any one or more of the preceding aspects, the inside diameter is between 1.0mm and 4.0mm.

In accordance with another exemplary aspect of the present disclosure, which may be used in combination with any one or more of the preceding aspects, the transducer is configured to emit an ultrasonic signal that is transmitted through the patient's vessel.

Aspects of the subject matter described herein may be useful alone or in combination with one or more other aspects described herein. In a seventh exemplary aspect of the present disclosure a vascular monitoring system includes a strap configured to transition from an open configuration to a closed configuration. The strap forming a collar when placed in the closed configuration, the collar configured to be positioned about a patient's vessel. The vascular monitoring system also includes a transducer coupled to the collar. Additionally, the transducer is configured to emit an ultrasonic signal that is transmitted through the patient's vessel.

In accordance with another exemplary aspect of the present disclosure, which may be used in combination with any one or more of the preceding aspects, the strap includes at least one closure structure configured to maintain the strap in the closed configuration.

In accordance with another exemplary aspect of the present disclosure, which may be used in combination with any one or more of the preceding aspects, the at least one closure structure includes a clamp, clasp, and band.

In accordance with another exemplary aspect of the present disclosure, which may be used in combination with any one or more of the preceding aspects, the at least one closure structure includes a prong and a sizing hole.

In accordance with another exemplary aspect of the present disclosure, which may be used in combination with any one or more of the preceding aspects, the strap includes a probe holder sized and shaped to receive the transducer.

In accordance with another exemplary aspect of the present disclosure, which may be used in combination with any one or more of the preceding aspects, the transducer is coupled to the strap through a friction fit with the probe holder.

In accordance with another exemplary aspect of the present disclosure, which may be used in combination with any one or more of the preceding aspects, the strap is made of at least one of implant grade liquid-silicon rubber ("LSR"), high-consistency silicone rubber ("HCR"), high density polyethylene ("HDPE"), Nusil 4750, Nusil 4840, and a thermoplastic.

Aspects of the subject matter described herein may be useful alone or in combination with one or more other aspects described herein. In an eighth exemplary aspect of the present disclosure a vascular strap includes a base portion and a saddle portion extending from the base portion. The saddle portion has a proximal end and two respective distal ends. The vascular strap also includes two respective band portions extending from the respective distal ends of the saddle portion. The saddle portion and the two respective band portions are sized and shaped to be positioned about a patient's vessel. Additionally, the vascular strap includes a probe holder formed within the base portion, the probe holder configured to receive a transducer.

In accordance with another exemplary aspect of the present disclosure, which may be used in combination with any one or more of the preceding aspects, the transducer is configured to emit an ultrasonic signal that is transmitted through the patient's vessel.

In accordance with another exemplary aspect of the present disclosure, which may be used in combination with any one or more of the preceding aspects, the vascular strap includes at least one eyelet that is adapted to be sutured to adjacent tissue to fixedly position the strap about the patient's vessel.

In accordance with another exemplary aspect of the present disclosure, which may be used in combination with any one or more of the preceding aspects, the probe holder includes a receptacle that is sized and shaped such that the transducer is coupled to the strap through a friction fit with the receptacle of the probe holder.

In accordance with another exemplary aspect of the present disclosure, which may be used in combination with any one or more of the preceding aspects, the collar is made of at least one of implant grade liquid-silicon rubber ("LSR") and high-consistency silicone rubber ("HCR") with a durometer between 40 and 80.

In accordance with another exemplary aspect of the present disclosure, which may be used in combination with any one or more of the preceding aspects, the saddle portion and the two respective band portions are sized such that when the vascular strap is closed to form a collar about a vessel, the inside diameter of the collar is between 1.0mm and 4.0mm.

In accordance with another exemplary aspect of the present disclosure, which may be used in combination with any one or more of the preceding aspects, the strap includes at least one closure structure configured to maintain the strap in the closed configuration.

It is accordingly an advantage of the present disclosure to improve accessibility of blood flow data.

It is another advantage of the present disclosure to improve the detection of blood flow to confirm vessel patency.

It is another advantage of the present disclosure to provide remote monitoring of blood flow at an anastomotic site.

It is yet a further advantage of the present disclosure to reduce the occurrence of free flap failure and serious adverse events due to insufficient blood flow in a free flap.

It is still another advantage of the present disclosure to provide a system, device and/or method for early detection of insufficient blood flow or circulation in a free flap.

Additional features and advantages of the disclosed vascular monitoring collar are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not have to have all of the advantages listed herein. Moreover, it should be noted that the language used in the specification has been principally selected for readability and instructional purposes, and not to limit the scope of the inventive subject matter.

### BRIEF DESCRIPTION OF THE FIGURES

Fig. 1 is a schematic view of a probe lead wire system according to an example embodiment of the present disclosure.
Fig. 2 is a perspective view of a vascular collar with a transducer coupled to the collar according to an example embodiment of the present disclosure.
Figs. 3A, 3B and 3C illustrate a vascular collar and transducer being positioned about a patient's vessel according to an example embodiment of the present disclosure.
Fig. 4A is a perspective view of another example vascular collar in an open configuration with a transducer coupled to the collar according to an example embodiment of the present disclosure.
Fig. 4B is a perspective view the vascular collar of Fig. 4A in the closed configuration according to an example embodiment of the present disclosure.
Figs. 5A, 5B and 5C illustrate a vascular collar and transducer being positioned about a patient's vessel according to an example embodiment of the present disclosure.
Fig. 6 is a perspective view of a vascular strap that forms a vascular collar according to an example embodiment of the present disclosure.
Fig. 7A is a perspective view of a vascular strap that forms a vascular collar according to an example embodiment of the present disclosure.
Fig. 7B is a front view of a vascular strap that forms a vascular collar according to an example embodiment of the present disclosure.
Fig. 7C is a cross-sectional view of the vascular strap of Fig. 7B along line 7C-7C according to an example embodiment of the present disclosure.
Figs. 8A and 8B illustrate a vascular strap and transducer being positioned about a patient's vessel to form a vascular collar according to an example embodiment of the present disclosure.
Figs. 9A and 9B illustrate a vascular strap and transducer being positioned about a patient's vessel to form a vascular collar according to an example embodiment of the present disclosure.

### DETAILED DESCRIPTION OF EXAMPLE EMBODIMENTS

As discussed above, a vascular monitoring collar is provided to improve the accessibility, detection and/or reliability of detecting blood flow to confirm vessel patency at an anastomotic site. While free flap surgeries have a history of success, highly undesirable consequences of a flap failure still remain a possibility. One of the main causes of flap failure is a lack of blood being supplied to the flap tissue after the free flap is reattached at the recipient site. Things that commonly disturb circulation in a flap include vascular occlusion, hemorrhage, or infection. When not enough blood is supplied to the flap tissue, tissue necrosis results. However, the vascular monitoring collar disclosed herein advantageously enables early detection of insufficient blood flow or circulation in a free flap so that it may be saved, or salvaged before tissue necrosis.

The above vascular monitoring collar may be used to monitor blood flow at the anastomotic site, upstream of the anastomotic site, or downstream of the anastomotic site to confirm vessel patency of a surgical procedure, such as a free flap transfer micro vascular reconstruction. The collar may be used in conjunction with a monitoring system in various environments such as a hospital operating room or a post-anesthesia care unit to detect blood flow and confirm vessel patency (either on-site or remotely) both intra-operatively and post-operatively. Free flap transfer may be used to recreate body parts from surgery due to cancer and injury using the patient's own tissue. Examples include breast reconstruction, tongue reconstruction, jaw and cheek reconstruction, hand and foot reconstruction after trauma injuries, etc. Typically, the microvascular anastomosis is the critical point of the surgery that determines the success of the flap. By providing monitoring capabilities of blood flow at an anastomotic site, the vascular monitoring collar disclosed herein allows early detection of low blood flow or lack of blood flow within the flap tissue thereby enabling a medical practitioner (e.g., a surgeon) to take corrective action before necrosis sets in and the free flap becomes unusable.

The vascular monitoring collar may be used in conjunction with a flow monitor system that includes multi-component probe systems, such as that described in PCT/US2018/061191 ("Vascular Monitoring System, Device and Method") the disclosure of which is incorporated herein by reference.

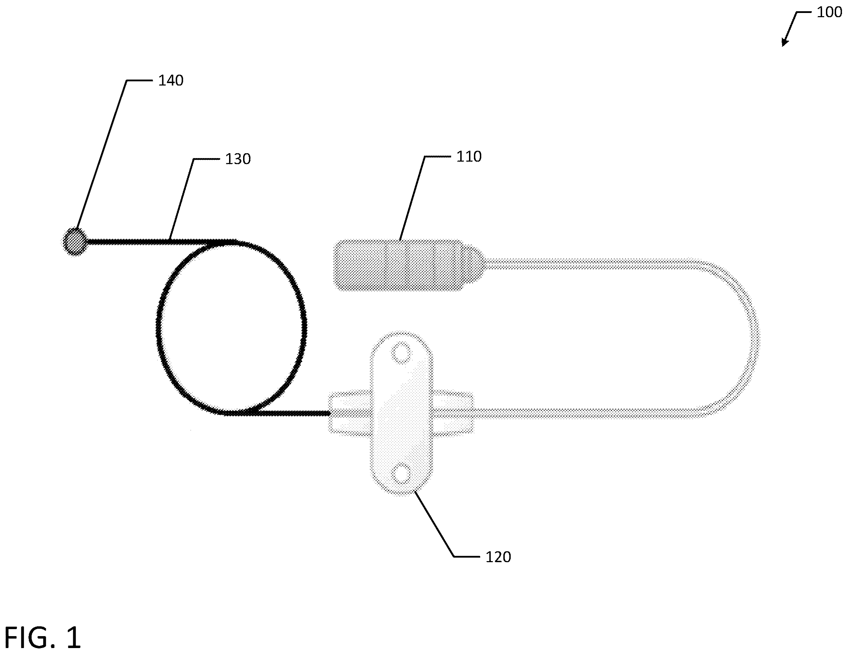

As illustrated in Fig. 1, a probe assembly 100 may include a probe connector 110 may be connected to a probe monitor system. The probe assembly 100 may also include a suture sleeve 120 that is configured for attachment (e.g., via sutures) to a patient's body or clothing. The suture sleeve 120 may be composed of medical grade material suitable for contact with human skin, for example, USP grade V or VI material. A variety of alternative approaches can be used to attach the probe assembly 100 or lead to the skin, including for instance the use of patches and bonding pads. The suture sleeve 120, bonding pad or alternative approaches may be attached to the skin in such a way that the force necessary to remove the pad or alternative approaches from the skin must be greater than the force necessary to remove the probe.

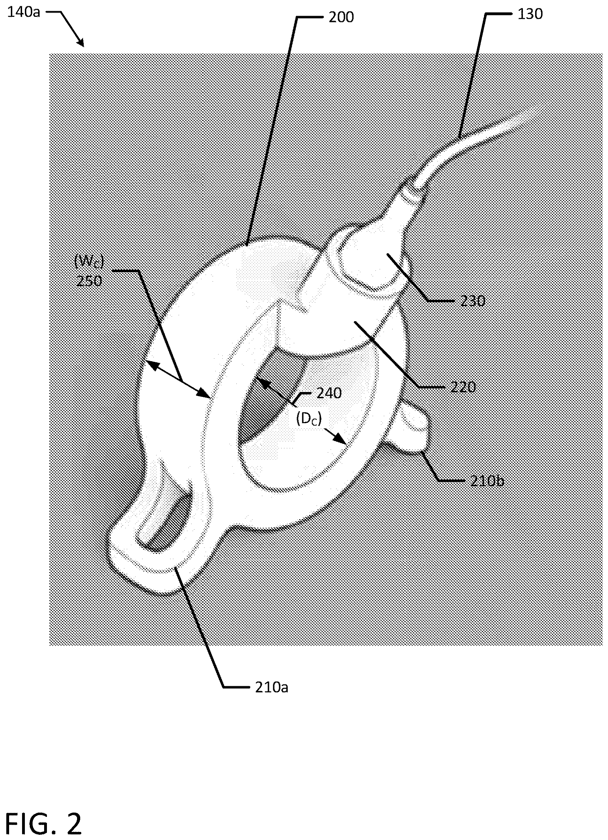

Extending from the probe connector 110 is a probe wire 130. At the end of the probe wire 130 is an "end-of-probe" component 140, such as a collar (see Figs. 2 to 8B) and/or the Doppler Probe or transducer that is coupled (e.g. press-fit) into the collar. In an example, the "end-of-probe" component 140 may include a transducer that is removably coupled to a separate collar. In another example, the "end-of-probe" component 140 may be a collar and transducer assembly (see Figs. 2 to 8B).

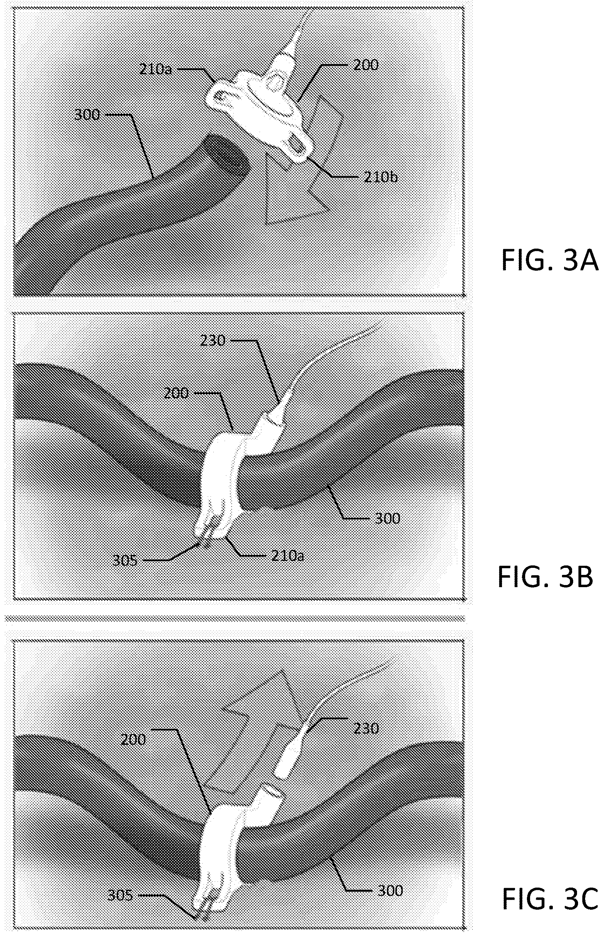

Fig. 2 illustrates an example "end-of-probe" component 140a. As illustrated in Fig. 2, a collar 200 may include eyelets 210a and 210b that provide a grasping surface for a clinician and that also allow the collar 200 to be anchored to adjacent tissue as further illustrated in Figs. 3B and 3C. The collar 200 also includes a probe holder 220 that is configured to receive a Doppler Probe or transducer 230. In an example, the Doppler Probe or transducer 230 may be press-fit into the probe holder 220. The probe holder 220 may include a receptacle that is configured to removably retain the Doppler Probe or transducer 230 at a predetermined distance and a predetermined angle with respect to a longitudinal axis of the collar 200. The receptacle of the probe holder 220 may have an octagonal or hexagonal profile. For example, the octagonal or hexagonal profile may provide multiple surfaces for frictional engagement with the Doppler Probe or transducer230. In an example, the angle of the Doppler Probe or transducer may be approximately 30 degrees from a flat end face of the collar 200 and thus 120 degrees from the longitudinal axis of the collar 200. In another example, the angle may be between 30 degrees and 60 degrees from the flat end face of the collar 200 and thus between 120 and 150 degrees from the longitudinal axis of the collar 200.

As illustrated in Fig. 2, the collar 200 has an inside diameter (D_{C}) 240 and a collar width (W_{C}) 250. The collar 200 may be sized and shaped (e.g., ring shaped) such that it fits on a similarly sized vessel (e.g., artery or vein). For example, the collar 200 may have an inside diameter (D_{C}) 240 between 1.0mm and 4.0mm. The collar width (W_{C}) 250 may be between 2.5mm and 5.0mm to provide stability on the vessel.

The collar 200 may be made from silicone, such as implant grade liquid-silicon rubber ("LSR") or high-consistency silicone rubber ("HCR"). The silicone may have a durometer between 40 and 80 (e.g., Shore A) and a tear strength between 240 and 350 ppi. The silicone described above allows the collar 200 to conform to the vessel surface. In other examples, the collar 200 may be made from high density polyethylene ("HDPE"). Alternatively, the collar 200 may be made from Nusil 4750, Nusil 4840, a thermoplastic, or the like. The collar 200 may be made from other flexible or malleable materials. In an example, the collar 200 is permanently implanted within the patient. Additionally, the collar may also be bioabsorbable.

As illustrated in Figs. 3A, 3B and 3C, the collar's 200 size and shape (e.g., ring shaped) is adapted such that the collar 200 fits on a similarly sized vessel (e.g., artery or vein). As discussed above, the collar may have an inside diameter (D_{C}) 240 between 1.0mm and 4.0mm. In an example, the inside diameter (Dc) 240 of the collar 200 may be provided in size increments of 0.5mm. It should be appreciated that the collar 200 may be sized and shaped to accommodate vessels (e.g., veins and arteries) typically encountered in microsurgical and vascular reconstructive procedures and are adapted for end-to-end anastomosis of such veins and arteries in the peripheral vascular system. For example, Figs. 3A and 3B illustrate the collar 200 being positioned over and advanced along a vessel 300 prior to an anastomosis. The collar 200 may be located near the anastomosis site such that the collar 200 is positioned at the anastomosis site, upstream of the anastomosis site or downstream of the anastomosis site. After the collar 200 is positioned in its intended location along the vessel 300, the collar 200 may be anchored to adjacent tissue by suturing the eyelets 210a and 210b to the adjacent tissue. Suturing the eyelets 210a and 210b to adjacent tissue may advantageously provide strain relief for Doppler Probe 230 removal as illustrated in Fig. 3C. Figs. 3B and 3C illustrate sutures 305 as the means of attaching the collar 200, and more specifically the eyelets 210a and 210b, to the adjacent tissue. It should be appreciated that other attachment means may be used such as staples, clips, etc.

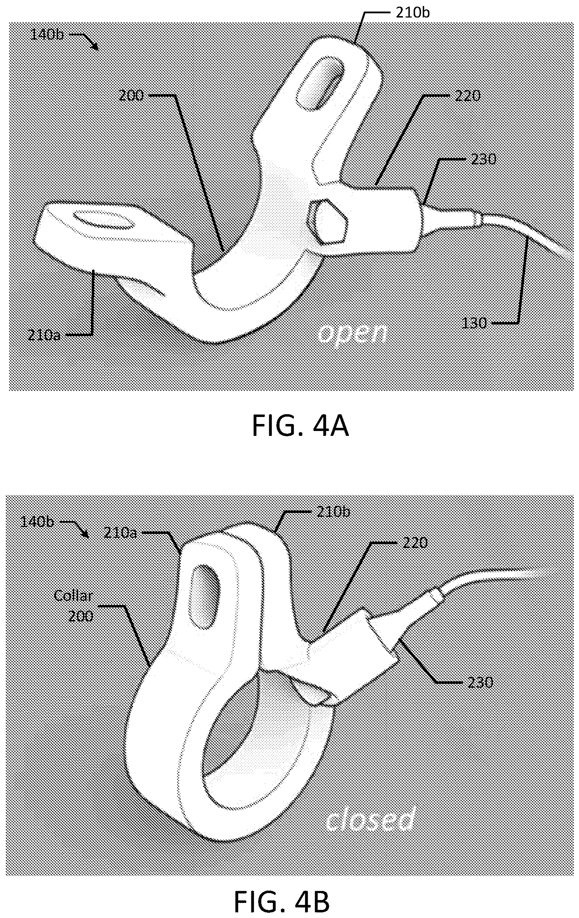

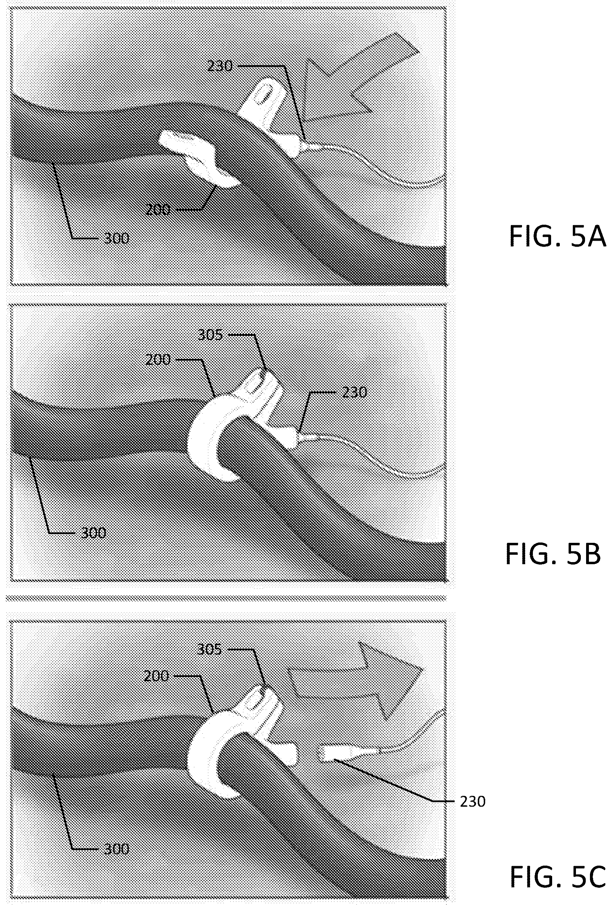

Figs. 4A and 4B illustrate another example "end-of-probe" component 140a and example collar 200. Fig. 4A illustrates the collar 200 in an open configuration while Fig. 4B illustrates the collar 200 in a closed configuration. Similar to the collar 200 illustrated in Fig. 2, the collar 200 illustrated in Figs. 4A and 4B may include eyelets 210a and 210b that provide a grasping surface for a clinician and that also allow the collar 200 to be anchored to adjacent tissue. For example, the clinician may grasp the eyelets 210a and/or 210b with tweezers, forceps, or other medical tool when positioning the collar 200. After the collar 200 is in place, the clinician may squeeze the eyelets 210a,b together to close the collar 200 and suture the two eyelets 210a,b together to maintain the collar 200 in the closed configuration (see Fig. 5B). After the collar 200 is closed about the vessel, the clinician may suture the eyelets 210a and/or 210b to nearby tissue. The collar 200 also includes a probe holder 220 that is configured to receive a Doppler Probe or transducer 230. In an example, the Doppler Probe or transducer 230 may be press-fit into the probe holder 220. The probe holder 220 may include a receptacle that is configured to removably retain the Doppler Probe or transducer 230 at a predetermined distance and a predetermined angle with respect to a longitudinal axis of the collar 200 when the collar 200 is in the closed configuration. In an example, the angle of the Doppler Probe or transducer 230 may be approximately 30 degrees from a flat end face of the collar 200 and thus 150 degrees from the longitudinal axis of the collar 200 when the collar 200 is in the closed configuration. In another example, the angle may be between 30 degrees and 60 degrees from the flat end face of the collar 200 and thus between 120 and 150 degrees from the longitudinal axis of the collar 200.

The collar 200 may be made from flexible or malleable materials that allow the collar 200 to transition between the open configuration and the closed configuration. In an example, the collar 200 is permanently implanted within the patient. Additionally, the collar 200 may also be bioabsorbable. For example, the collar 200 illustrated in Figs. 4A, 4B, 5A, 5B and 5C may have the same material properties as the collar 200 illustrated in Figs. 2, 3A, 3B and 3C.

As illustrated in Fig. 4A, the collar 200 starts in an open configuration and may be positioned along a vessel even if the vessel has not been severed or cut for an anastomosis. For example, the collar 200 may be positioned along an uncut vessel to monitor blood flow through that vessel. The collar 200 illustrated in Figs. 4A and 4B may also be advanced along a vessel prior to an anastomosis or after an anastomosis has been completed, which advantageously provides flexibility during a surgical operation. Similar to the collar 200 illustrated in Figs. 2, 3A, 3B and 3C, the collar 200 of Figs. 4A and 4B may be located near the anastomosis site such that it is positioned at the anastomosis site, upstream of the anastomosis site or downstream of the anastomosis site.

Figs. 5A, 5B and 5C illustrate positioning the collar 200 on a vessel 300. The collar 200 may be sized and shaped (e.g., clip shaped) such that the collar 200 fits on a similarly sized vessel 300 (e.g., artery or vein). For example, the collar 200 may have an inside diameter (D_{C}) 240 similar to that of collar 200 of Fig. 2, when in the closed configuration, between 1.0mm and 4.0mm. In an example, the inside diameter (D_{C}) 240 of the collar 200 in the closed position may be provided in size increments of 0.5mm. It should be appreciated that the collar 200 may be sized and shaped to accommodate vessels (e.g., veins and arteries) typically encountered in microsurgical and vascular reconstructive procedures and are adapted for end-to-end anastomosis of such veins and arteries in the peripheral vascular system. After the collar 200 is positioned in its intended location along the vessel 300, the collar 200 may closed by suturing the eyelets 210a,b together such that the collar 200 remains in the closed configuration. The collar 200 may also be anchored to adjacent tissue by suturing the eyelets 210a and/or 210b to the adjacent tissue. Suturing the eyelets 210a,b to adjacent tissue may advantageously provide strain relief for Doppler Probe 230 removal as illustrated in Fig. 5C. Figs. 5B and 5C illustrate sutures 305 as the means of maintaining the collar 200 in the closed configuration. It should be appreciated that other attachment means may be used such as staples, clips, etc. to maintain the collar 200 in the closed configuration.

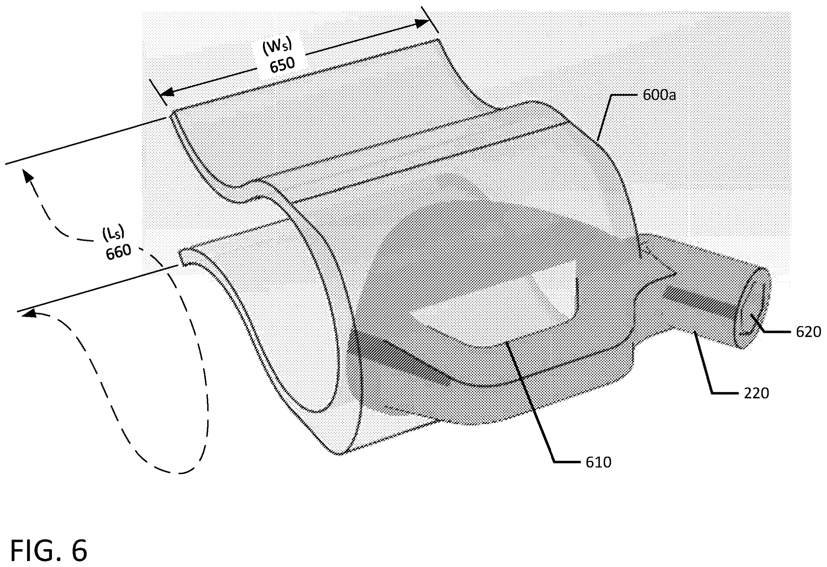

Fig. 6 illustrates another embodiment of a collar or strap 600a. For example, as illustrated in Fig. 6, the strap 600a may include an eyelet(s) 610 that provides a grasping surface for a clinician and that also allows the collar or strap 600a to be anchored to adjacent tissue. The collar or strap 400 also includes a probe holder 220 that is configured to receive a Doppler Probe or transducer 230. Similar to the embodiments described in Figs. 2 to 5C, the Doppler Probe or transducer 230 may be press-fit into the probe holder 220. As mentioned above, the probe holder 220 may include a receptacle 620 that is configured to removably retain the Doppler Probe or transducer 230 at a predetermined distance and a predetermined angle with respect to a longitudinal axis of the collar or strap 600a when the strap 600a is closed around a vessel. The receptacle 620 of the probe holder 220 may be sized and shaped similar to the probe holder illustrated in Figs. 2 to 5C. For example, the probe holder 220 may have an octagonal or hexagonal profile that provides multiple surfaces for frictional engagement with the Doppler Probe or transducer 230. In an example, the angle of the Doppler Probe or transducer 230 may be approximately 30 degrees to 60 degrees from a flat end face of the collar or strap 600a and thus 120 degrees to 150 degrees from the longitudinal axis of the collar formed by the strap 600a when the strap 600a is closed around the vessel.

The collar or strap 600a may be made from high-density polyethylene ("HDPE"). In an example, the strap 600a may be made from silicone such as implant grade liquid-silicon rubber ("LSR") or high-consistency silicone rubber ("HCR"). The silicone may have a durometer between 40 and 80 (e.g., Shore A) and a tear strength between 240 and 350 ppi. The silicone described above allow the collar or strap to conform to the vessel surface while providing a robust material that can withstand the stresses associated with closing the strap 600a around a vessel. In other examples, the strap 600a may be made from Nusil 4750, Nusil 4840, a thermoplastic, or the like. The strap 600a may be made from other flexible or malleable materials such that the strap 600a is adapted to wrap around a patient's vessel. In an example, the strap 600a is permanently implanted within the patient and may be bioabsorbable.

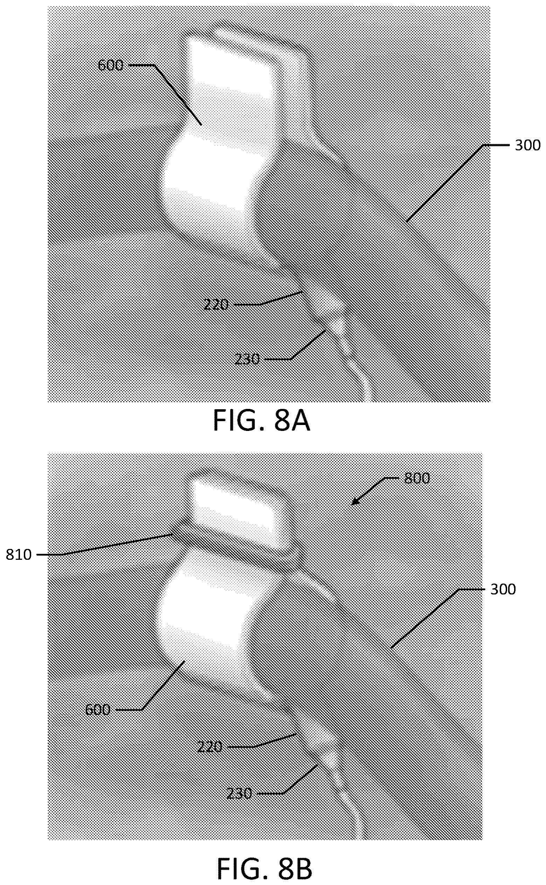

Once the strap 600a is wrapped around a patient's vessel and maintained in its closed position, the strap 600a may resemble a closed collar. The strap 600a has a strap width (W_{S}) 650 and a strap length (L_{S}) 660. The strap width (W_{S}) may be between 2.5mm and 5.0mm to provide stability on the vessel. The strap length (L_{S}) 660 may be sufficiently long such that the strap 600a can be wrapped around a vessel and also have sufficient length for closure (see Figs. 8A and 8B). For example, the strap 600a may be sized and shaped such that when closed, the strap 600a forms a collar that fits on a similarly sized vessel (e.g., artery or vein). For example, the collar formed by the closed strap 600a may have an inside diameter between 1.0mm and 4.0mm. In an example, the strap 600a may be provided in increments of approximately 1.5mm to accommodate different vessel sizes (e.g., vessels sizes that differ in increments of approximately 0.5mm). It should be appreciated that the strap 600a may be sized and shaped to accommodate vessels (e.g., veins and arteries) typically encountered in microsurgical and vascular reconstructive procedures and are adapted for end-to-end anastomosis of such veins and arteries in the peripheral vascular system.

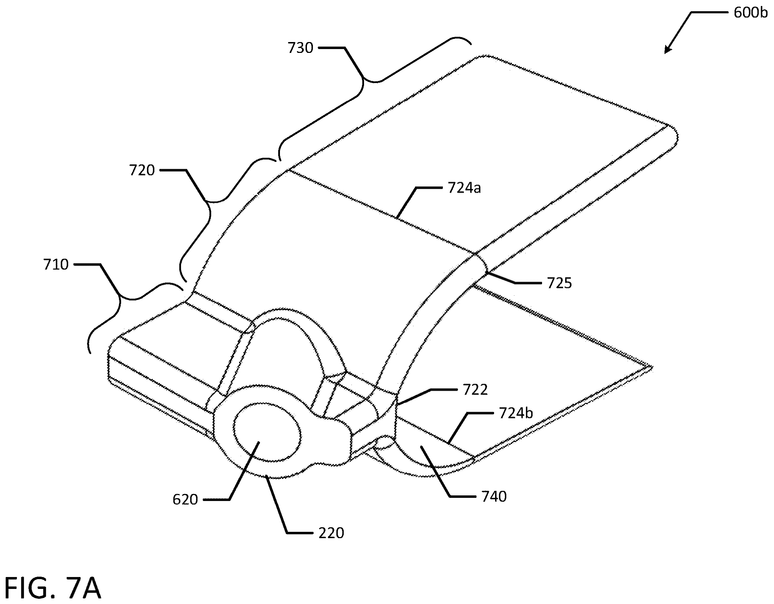

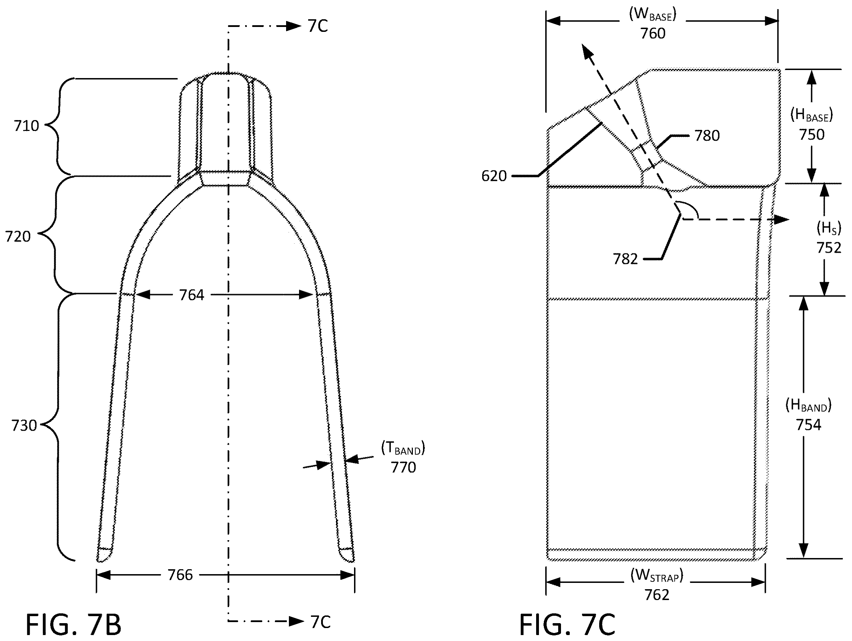

Figs. 7A, 7B and 7C illustrate another example embodiment of a strap 600b. The strap 600b may include a base portion 710, a saddle portion 720 and a band portion 730. The probe holder 220 may be formed as part of the base portion 710, which provides stability to the strap 600b and also provides a grasping surface for a clinician when manipulating and positioning the strap 600b. The saddle portion 720 has a proximal end 722 and two respective distal ends 724a,b. The saddle portion 720 may extend from the base portion 710 at the saddle portion's proximal end 722. Extending from the saddle portion 720 on each end is a respective band portion 730. For example, each respective band portion 730 may extend from the respective distal end 724a,b of the saddle portion 720.

The saddle portion 720 and the respective band portions 730 may meet at a joint 725 (e.g., the respective distal ends 724a,b of the saddle portion 720). When the strap 600b is spread into an open-most configuration, a first end of the strap 600b would be a band portion 730, followed by a first part of the saddle portion 720 and the base portion 710, then the strap 600b would continue to a second part of the saddle portion 730 and another respective band portion 730.

In an example, the saddle portion 720 extends outward from the base portion 710 and forms a contact surface 740 for a portion of a vessel. The contact surface 740 may be shaped like an inverted or upside-down saddle that creates a bowl-like or basin-like surface. For example, the saddle portion 720 may be flexible while maintaining enough rigidity to create a pre-formed contact surface 740. Alternatively, the saddle portion 720 and the band portion 730 may be sufficiently flexible and elastic such that the strap 600b would sit flat on a horizontal surface when the contact surface 740 is adjacent to the horizontal surface.

As illustrated in Figs. 7B and 7C, the base portion 710 has a height (H_{BASE}) 750 and a width (W_{BASE}) 760. The height (H_{BASE}) 750 may be approximately 2.25mm and the width (W_{BASE}) 760 may be between 2.5mm and 5.0mm. Wider base portions 710 may be implemented to provide additional stability on the vessel.

Additionally, the saddle portion 720 has a height (Hs) 752, which may be approximately 2.65mm. The distance 764 between each end of the saddle portion 720 (e.g., at the joint 725) may be approximately 4.0mm. The band portion 730 has a height (H_{BAND}) 754, which may be approximately 6.0mm. When the strap 600b is in a relaxed position (as illustrated in Fig. 7B), especially with a saddle portion 720 that retains its shape, the distance 766 between each end of the band portions 730 may be approximately 5.0mm.

The band portion may have a wall thickness (T_{BAND}) 770 between approximately 0.1mm and approximately 0.3mm. The wall thickness (T_{BAND}) 770 may be selected and configured based on a closure mechanism for the strap 600b. For example, different closure clamps may be compatible with different wall thicknesses. Additionally, the wall thickness (T_{BAND}) 770 may be selected to increase or decrease the flexibility, rigidity and/or durability of the strap 600b. The strap 600b may have a width (W_{STRAP}) 762 at the end of the band portion 730 of approximately 2.5mm to 5.0mm. Similar to the wall thickness (T_{BAND}) 770, the width (W_{STRAP}) 762 may be selected to increase or decrease the flexibility, rigidity and/or durability of the strap 600b. Additionally, the width (W_{STRAP}) 762 may be selected and configured based on a closure mechanism for the strap 600b. For example, different closure clamps may be compatible with different strap widths.

The dimensions of the saddle portion 720 and the band portions 730 may be adjusted for different vessel sizes. For example, the band portion may have sufficient height to provide an adequate closure surface after the strap 600b is closed around a vessel having a vessel diameter between 1.0mm and 4.0mm. It should be appreciated that the strap 600ab may be sized and shaped to accommodate vessels (e.g., veins and arteries) typically encountered in microsurgical and vascular reconstructive procedures and are adapted for end-to-end anastomosis of such veins and arteries in the peripheral vascular system.

As illustrated in Fig. 7C, which is a cross-sectional view about line 7C-7C of Fig. B, the receptacle 620 of the probe holder 220 may have a conical profile with a cylindrical transition region 780. The diameter of the cylindrical transition region 780 may be between 0.015 inches and 0.030 inches (e.g., 0.38mm and 0.76mm). Cylindrical transition regions 780 with smaller diameters may provide a tighter grip or squeeze on a corresponding Doppler probe or transducer 230. The probe holder 220 may be oriented at an angle 782 that is between 120 and 150 degrees from the longitudinal axis of the collar formed by the closed strap 600b.

Figs. 8A and 8B illustrate an example of positioning a strap, such as strap 600a or strap 600b about a vessel 300. Straps 600a and 600b may be generally referred to strap 600 hereafter. The strap 600 illustrated in Figs. 8A and 8B may include each of the features of the strap 600a described in Fig. 6, each of the features of strap 600b or a combination thereof. As illustrated in Fig. 8B, a strap assembly 800 may include a clamp 810, clasp, band or other closure mechanism that maintains the strap 600 in a closed configuration about a vessel 300 such that the strap 600 forms a collar around the vessel 300. For example, Figs. 8A and 8B illustrate the strap 600 being wrapped around a vessel 300 to form a collar. The collar formed by the strap 600 may be located near the anastomosis site such that it is positioned at the anastomosis site, upstream of the anastomosis site or downstream of the anastomosis site. After the strap 600 is wrapped around the vessel 300 and maintained in its closed orientation (e.g., such that the strap 600 forms a collar) and is positioned in its intended location along the vessel 300, the collar formed by the strap 600 may be anchored to adjacent tissue by suturing an eyelet(s) (see Fig. 6) to the adjacent tissue. Similar to the embodiments described in Figs. 3C and 5C, suturing the eyelets to adjacent tissue may advantageously provide strain relief for Doppler Probe removal.

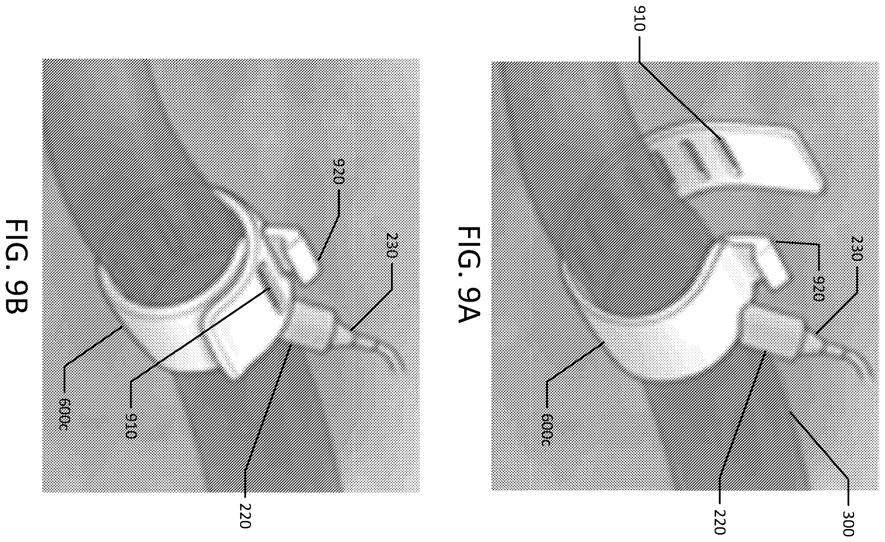

Figs. 9A and 9B illustrate another example embodiment of a strap 600c with a different closure mechanism than the clamp, clasp or band illustrated in Fig. 8B. For example, the strap 600c may include multiple sizing holes 910 that are spaced along the strap 600c and are adapted to maintain the strap 600c in a closed configuration when fit over a closure prong 920. For example, the sizing holes 910 may be sized and shaped such that they may be press-fit over the closure prong 920. The sizing holes 910 may be spaced along the strap 600c with a spacing of approximately 1.5mm between each hole to accommodate different vessel sizes (e.g., vessels sizes that differ in increments of approximately 0.5mm). The spacing between each sizing hole 910 may instead be 1.0mm or some other interval to accommodate different intervals of vessel sizes.

As discussed above, the straps 600a, 600b and 600c described herein may be sized and shaped for specific vessel sizes such that one strap is configured for vessels between 1.0 and 2.0mm, another strap is configured for vessels between 2.0 and 3.0mm, and a different strap is configured for vessels between 3.0 and 4.0mm. In the case where there are different strap sizes or lengths adapted for different vessel sizes, the sizing holes 910 may be positioned with a tighter spacing such that the strap can be adjusted to fit around a vessel between 1.0 and 2.0mm in diameter in increments of 0.2mm (e.g., the sizing holes 910 may be configured such that the strap can be adjusted to form a collar that has an inside diameter of 1.0mm, 1.2mm, 1.4mm, 1.6mm, 1.8mm and 2.0mm). It should be appreciated that the strap 600c may be sized and shaped and the sizing holes 910 may be positioned to accommodate vessels (e.g., veins and arteries) typically encountered in microsurgical and vascular reconstructive procedures and are adapted for end-to-end anastomosis of such veins and arteries in the peripheral vascular system.

The prong 920 may include a flange or lip that is configured to maintain the strap 600c in the closed configuration. For example, a sizing hole 910 may be positioned over and press-fit over the prong 920 such that the prong 920 is pushed through the sizing hole 910. The material of the strap may allow the sizing hole to expand and flex to fit over the flange or lip of the prong 920 before relaxing back to its original shape. After the prong 920 is pushed through the sizing hole 910, the flange or lip is adapted to prevent the strap 600c from unwinding to an open position. For example, the flange or lip may be sized and shaped such that the forces associated with the strap's tendency to relax back to its open position are insufficient to cause the sizing hole 910 to expand and flex to fit back over the flange or lip of the prong 920. The material of the strap 600c and the geometry of both the sizing hole 910 and prong are configured such that a clinician can manipulate the strap 600c between an open configuration and closed configuration while also preventing the strap 600c from opening without clinician intervention.

Similar to the strap illustrated in Figs. 8A and 8B, the strap illustrated in Figs. 9A and 9B may include each of the features of the strap described in Fig. 6 or Figs. 7A-7C. Additionally, the straps illustrated in Figs. 6, 7A-7C, 8A, 8B, 9A and 9B may be configured and arranged such that when in a closed configuration, the straps form a collar that is oriented similarly as the collars illustrated in Figs. 2, 3A, 3B, 3C, 4B, 5B and 5C. For example, the probe holder may include a receptacle that is configured to removably retain the Doppler Probe or transducer at a predetermined distance and a predetermined angle with respect to a longitudinal axis of the collar formed by the strap when the strap is in the closed configuration (e.g., the angle of the Doppler Probe or transducer may be approximately 30 degrees from a flat end face of the collar and thus 120 degrees from the longitudinal axis of the collar formed by the strap when the strap is in the closed configuration). In another example, the angle may be between 30 degrees and 60 degrees from the flat end face of the strap 600 and thus between 120 and 150 degrees from the longitudinal axis of the collar formed by the closed strap 600.

Sensing device(s), such as the Doppler Probe or transducer inserted into the collar enables a medical practitioner (e.g., surgeon) to monitor and analyze the blood flow and/or blood velocity to determine the success of the surgery and/or to confirm vessel patency.

Any transducer suitable for ultrasonic Doppler monitoring may be used with the collar. In an example embodiment, the Doppler Probe or transducer is made of an approved implantable material such as HDPE or silicone. In another example, the transducer 230 comprises a piezoelectric crystal. The transducer 230 may be any size conforming to the dimensions of a corresponding probe holder used on the collar. For example, a circular transducer 230 is suitable to be received by a receptacle having its internal surface circular in shape. In another example, the receptacle 620 formed by the probe holder may be octagonal or hexagonal (see Fig. 3A) to provide a tighter friction fit with the Doppler probe or transducer tip. The transducer 230 may be a circular piezoelectric crystal being between about 0.5 mm to about 1 mm in size. In one example, the Doppler Probe or transducer 230 includes a tip with a circular piezoelectric crystal being between about 0.5 mm to about 1 mm in size, a Teflon-coated coax wire and a metal connector.

The Doppler Probes coupled to the collars or straps disclosed herein may be adapted to detect blood flow at the anastomotic site and confirm vessel patency intra-operatively and post-operatively at the anastomotic site. For example, blood flow can be detected post-operatively for up to approximately 14 days.

The many features and advantages of the present disclosure are apparent from the written description, and thus, the appended claims are intended to cover all such features and advantages of the disclosure. Further, since numerous modifications and changes will readily occur to those skilled in the art, the present disclosure is not limited to the exact construction and operation as illustrated and described. Therefore, the described embodiments should be taken as illustrative and not restrictive, and the disclosure should not be limited to the details given herein but should be defined by the following claims and their full scope of equivalents, whether foreseeable or unforeseeable now or in the future.

The following embodiments are further referred to in the present disclosure:
1. A vascular monitoring system comprising:
   a collar configured to be positioned about a patient's vessel; and
   a transducer coupled to the collar, the transducer configured to emit an ultrasonic signal that is transmitted through the patient's vessel.
2. The monitoring system of embodiment 1, wherein the collar includes at least one eyelet that is adapted to be sutured to adjacent tissue to fixedly position the collar about the patient's vessel.
3. The monitoring system of any of embodiments 1 or 2, wherein the collar includes a probe holder sized and shaped to receive the transducer.
4. The monitoring system of embodiment 3, wherein the transducer is coupled to the collar through a friction fit with the probe holder.
5. The monitoring system of any of embodiments 1 to 4, wherein the collar is made of at least one of implant grade liquid-silicon rubber (LSR) and high-consistency silicone rubber (HCR) with a durometer between 40 and 80.
6. The monitoring system of any of embodiments 1 to 5, wherein the collar is configured to be positioned about an anastomosis site of the patient's vessel.
7. The monitoring system of any of embodiments 1 to 5, wherein the collar is configured to be positioned at a location that is one of upstream of an anastomosis site of the patient's vessel and downstream of the anastomosis site of the patient's vessel.
8. The monitoring system of any of embodiments 1 to 7, wherein the transducer is removably coupled to the collar.
9. A vascular collar comprising:
   a cylindrical body portion with an opening, the opening having an inside diameter sized and shaped to be positioned about a patient's vessel;
   a probe holder, the probe holder configured to receive a transducer; and
   at least one mounting tab.
10. The vascular collar of embodiment 9, wherein the inside diameter is between 1.0mm and 4.0mm.
11. The vascular collar of any of embodiments 9 and 10, wherein the transducer is configured to emit an ultrasonic signal that is transmitted through the patient's vessel.
12. A vascular monitoring system comprising:
   a collar configured to be positioned about a patient's vessel, the collar configured to transition from an open configuration to a closed configuration; and
   a transducer coupled to the collar, the transducer configured to emit an ultrasonic signal that is transmitted through the patient's vessel.
13. The system of embodiment 12, wherein the collar includes at least one closure structure configured to maintain the collar in the closed configuration.
14. The system of embodiment 13, wherein the at least one closure structure includes a first eyelet and a second eyelet.
15. The system of any of embodiments 13 and 14, wherein the at least one closure structure is adapted to be sutured to adjacent tissue to fixedly position the collar about the patient's vessel.
16. The monitoring system of any of embodiments 12 to 15, wherein the collar includes a probe holder sized and shaped to receive the transducer.
17. The monitoring system of embodiment 16, wherein the transducer is coupled to the collar through a friction fit with the probe holder.
18. The monitoring system of any of embodiments 12 to 17, wherein the collar is made of at least one of implant grade liquid-silicon rubber (LSR) and high-consistency silicone rubber (HCR) with a durometer between 40 and 80.
19. The monitoring system of any of embodiments 12 to 18, wherein the collar is configured to be positioned about an anastomosis site of the patient's vessel.
20. The monitoring system of any of embodiments 12 to 18, wherein the collar is configured to be positioned at a location that is one of upstream of an anastomosis site of the patient's vessel and downstream of the anastomosis site of the patient's vessel.
21. The monitoring system of any of embodiments 12 to 20, wherein the transducer is removably coupled to the collar.
22. A vascular collar comprising:
   a body portion that is configured to transition from an open configuration to a closed configuration, wherein the body portion has an opening in the closed configuration, the opening having an inside diameter sized and shaped to be positioned about a patient's vessel;
   a probe holder, the probe holder configured to receive a transducer; and
   at least one mounting tab.
23. The vascular collar of embodiment 22, wherein the mounting tab includes a closure feature that is adapted to retain the collar in the closed configuration after transitioned from the open configuration to the closed configuration.
24. The vascular collar of any of embodiments 22 and 23, wherein the body portion is made from a flexible/malleable material that allows the body portion to transition from the open configuration to the closed configuration when a closure force is applied to the collar.
25. A vascular monitoring system comprising:
   a strap configured to be positioned about a patient's vessel;
   a clasp configured to maintain the strap in a closed configuration about the patient's vessel; and
   a transducer coupled to the strap, the transducer configured to emit an ultrasonic signal that is transmitted through the patient's vessel.
26. The monitoring system of embodiment 25, wherein the strap includes at least one eyelet that is adapted to be sutured to adjacent tissue to fixedly position the strap about the patient's vessel.
27. The monitoring system of any of embodiments 25 and 26, wherein the strap includes a probe holder sized and shaped to receive the transducer.
28. The monitoring system of embodiment 27, wherein the transducer is coupled to the strap through a friction fit with the probe holder.
29. The monitoring system of any of embodiments 25 to 28, wherein the strap is made of at least one of at least one of implant grade liquid-silicon rubber (LSR), high-consistency silicone rubber (HCR), HDPE, Nusil 4750, Nusil 4840, and a thermoplastic.
30. The monitoring system of any of embodiments 25 to 29, wherein the strap, in its closed configuration, is configured to be positioned about an anastomosis site of the patient's vessel.
31. The monitoring system of any of embodiments 25 to 29, wherein the strap, in its closed configuration, is configured to be positioned at a location that is one of upstream of an anastomosis site of the patient's vessel and downstream of the anastomosis site of the patient's vessel.
32. The monitoring system of any of embodiments 25 to 31, wherein the transducer is removably coupled to the collar.
33. A vascular strap comprising:
   an elongate strap body having a first end and second end;
   a plurality of sizing holes positioned along the strap body starting near the first end;
   a closure prong positioned adjacent the second end of the strap body, wherein
      the closure prong is sized and shaped to press-fit through a sizing hole of the plurality of sizing holes,
      the closure prong is configured to maintain the vascular strap in a closed configuration when press-fit through the sizing hole, and
      the closed configuration forms a cylindrical shape having an inside diameter sized and shaped to be positioned about a patient's vessel;
   a probe holder, the probe holder configured to receive a transducer; and
   at least one mounting tab.
34. The vascular strap of embodiment 33, wherein the inside diameter is between 1.0mm and 4.0mm.
35. The vascular strap of any of embodiments 33 and 34, wherein the transducer is configured to emit an ultrasonic signal that is transmitted through the patient's vessel.
36. A vascular monitoring system comprising:
   a strap configured to transition from an open configuration to a closed configuration, the strap forming a collar when placed in the closed configuration, the collar configured to be positioned about a patient's vessel; and
   a transducer coupled to the collar, the transducer configured to emit an ultrasonic signal that is transmitted through the patient's vessel.
37. The monitoring system of embodiment 36, wherein the strap includes at least one closure structure configured to maintain the strap in the closed configuration.
38. The system of embodiment 37, wherein the at least one closure structure includes a clamp, clasp, and band.
39. The system of embodiment 37, wherein the at least one closure structure includes a prong and a sizing hole.
40. The monitoring system of any of embodiments 36 to 39, wherein the strap includes a probe holder sized and shaped to receive the transducer.
41. The monitoring system of embodiment 40, wherein the transducer is coupled to the strap through a friction fit with the probe holder.
42. The monitoring system of any of embodiments 36 to 41, wherein the strap is made of at least one of implant grade liquid-silicon rubber (LSR), high-consistency silicone rubber (HCR), HDPE, Nusil 4750, Nusil 4840, and a thermoplastic.
43. A vascular strap comprising:
   a base portion;
   a saddle portion extending from the base portion, the saddle portion having a proximal end and two respective distal ends; and
   two respective band portions extending from the respective distal ends of the saddle portion, wherein the saddle portion and the two respective band portions are sized and shaped to be positioned about a patient's vessel; and
   a probe holder formed within the base portion, the probe holder configured to receive a transducer.
44. The vascular strap of embodiment 43, wherein the transducer is configured to emit an ultrasonic signal that is transmitted through the patient's vessel.
45. The vascular strap of any of embodiments 43 and 44, wherein the vascular strap includes at least one eyelet that is adapted to be sutured to adjacent tissue to fixedly position the strap about the patient's vessel.
46. The vascular strap of any of embodiments 43 to 45, wherein the probe holder includes a receptacle that is sized and shaped such that the transducer is coupled to the strap through a friction fit with the receptacle of the probe holder.
47. The vascular strap of any of embodiments 43 to 46, wherein the collar is made of at least one of implant grade liquid-silicon rubber (LSR) and high-consistency silicone rubber (HCR) with a durometer between 40 and 80.
48. The vascular strap of any of embodiments 43 to 47, wherein the saddle portion and the two respective band portions are sized such that when the vascular strap is closed to form a collar about a vessel, the inside diameter of the collar is between 1.0mm and 4.0mm.
49. The vascular strap of any of embodiments 43 to 48, wherein the strap includes at least one closure structure configured to maintain the strap in the closed configuration.

## Claims

1. A vascular monitoring system comprising:
a strap (600c) configured to be positioned about a patient's vessel (300); and
a transducer (230) coupled to the strap (600c), the transducer (230) configured to emit an ultrasonic signal that is transmitted through the patient's vessel (300),
wherein the strap (600c) includes multiple sizing holes (910) that are spaced along the strap (600c) and are adapted to maintain the strap (600c) in a closed configuration when fit over a closure prong (920).

2. The monitoring system of claim 1, wherein the plurality of holes (910) are spaced along the strap (600c).

3. The monitoring system of claim 1, wherein the strap (600c) is configured to be positioned about an anastomosis site of the patient's vessel (300).

4. The monitoring system of claim 3, wherein the strap (600c) is configured to be positioned at a location that is one of upstream of an anastomosis site of the patient's vessel (300) and downstream of the anastomosis site of the patient's vessel (300).

5. The monitoring system of claim 1, wherein the transducer (230) is removably coupled to the strap (600c).

6. The monitoring system of claim 2, wherein the plurality of holes (910) are configured to maintain the strap (600c) in a closed configuration.

7. The monitoring system of claim 1, wherein the plurality of holes (910) are spaced along the strap (600c) with a spacing of 1.0 mm to 2 mm between each hole.

8. The monitoring system of claim 1, wherein the strap (600c) is configured to transition from an open configuration to a closed configuration.

9. The monitoring system of claim 1, wherein the closure prong (920) includes a lip configured to maintain the strap (600c) in a closed configuration.

10. The monitoring system of claim 1, wherein the vascular monitor system further comprises a probe holder (220).

11. The monitoring system of claim 10, wherein the transducer (230) is coupled to the strap (600c) through a friction fit with the probe holder (220).

12. The monitoring system of claim 1, wherein the strap (600c) is made of at least one of implant grade liquid-silicon rubber (LSR) and high-consistency silicone rubber (HCR) with a durometer between 40 and 80.

13. The monitoring system of claim 1, further comprising a receptacle (620) configured to receive the transducer (230) and removably retain the transducer (230).

14. The monitoring system of claim 13, wherein the receptacle (620) has a conical profile.

15. The monitoring system of claim 11, wherein the probe holder (220) has a cylindrical transition region (780).
